(19) Europäisches Patentamt
European Patent Office
Office européen des brevets

(11) **EP 2 077 332 B1**

(12) **EUROPEAN PATENT SPECIFICATION**

(45) Date of publication and mention
of the grant of the patent:
**09.03.2016 Bulletin 2016/10**

(51) Int Cl.:
**C12Q 1/44** (2006.01)

(21) Application number: **08173028.5**

(22) Date of filing: **15.07.2005**

(54) **Secretory phospholipase A2 (sPLA2) as prognostic and diagnostic marker for cardiovascular diseases**

Sezernierte Phospholipase A2 (SPLA2) als Marker für die Prognose und Diagnose kardiovaskulärer Erkrankungen

Phospholipase A2 sécretoire comme marqueur pronostique et diagnostique de maladies cardiovasculaires

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.07.2004 EP 04291825**

(43) Date of publication of application:
**08.07.2009 Bulletin 2009/28**

(62) Document number(s) of the earlier application(s) in accordance with Art. 76 EPC:
**05766051.6 / 1 769 083**

(73) Proprietors:
• **INSERM (Institut National de la Santé et de la Recherche Médicale)**
**75654 Paris Cedex 13 (FR)**
• **L'Assistance Publique - Hôpitaux de Paris**
**75001 Paris (FR)**
• **Université Paris Diderot - Paris 7**
**75205 Paris Cedex 13 (FR)**

(72) Inventors:
• **Mallat, Ziad**
**78580 Herbeville (FR)**
• **Tedgui, Alain**
**75001 Paris (FR)**
• **Steg, Philippe Gabriel**
**75008 Paris (FR)**
• **Freyssinet, Jean-Marie**
**67000 Strasbourg (FR)**
• **Benessiano, Joëlle**
**75017 PARIS (FR)**

(74) Representative: **Icosa**
**83 avenue Denfert-Rochereau**
**75014 Paris (FR)**

(56) References cited:
**US-A1- 2003 219 849**

• **KUGIYAMA K ET AL: "Circulating levels of secretory type II phospholipase A2 predict coronary events in patients with coronary artery disease" CIRCULATION, AMERICAN HEART ASSOCIATION, DALLAS, TX, US, vol. 100, no. 12, 12 September 1999 (1999-09-12), pages 1280-1284, XP002258591 ISSN: 0009-7322**
• **LIU PING-YEN ET AL: "Prognostic value and the changes of plasma levels of secretory type II phospholipase A2 in patients with coronary artery disease undergoing percutaneous coronary intervention." EUROPEAN HEART JOURNAL. OCT 2003, vol. 24, no. 20, October 2003 (2003-10), pages 1824-1832, XP002301731 ISSN: 0195-668X**
• **DATABASE BIOSIS [Online] BIOSCIENCES INFORMATION SERVICE, PHILADELPHIA, PA, US; 2002, KOROTAEVA A A ET AL: "The level of secretory phospholipase A2 in the blood serum as a predictor of restenosis after coronary angioplasty" XP002301732 Database accession no. PREV200200456641 & TERAPEVTICHESKII ARKHIV, vol. 74, no. 4, 2002, pages 12-15, ISSN: 0040-3660**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **NIESSEN HANS W M ET AL: "Type II secretory phospholipase A2 in cardiovascular disease: A mediator in atherosclerosis and ischemic damage to cardiomyocytes?" CARDIOVASCULAR RESEARCH, vol. 60, no. 1, 15 October 2003 (2003-10-15), pages 68-77, XP002306886 ISSN: 0008-6363**
- **SCHAEFER R ET AL: "SERUM PHOSPHOLIPASE A AND PROGNOSIS IN INTENSIVE CARE PATIENTS", JOURNAL OF CLINICAL CHEMISTRY AND CLINICAL BIOCHEMISTRY, WALTER DE GRUYTER GMBH & CO. KG, GERMANY, vol. 28, no. 4, 1 January 1990 (1990-01-01), pages 253-254, XP009121022, ISSN: 0340-076X**

- **LANGTON S R ET AL: "Serum phospholipase A2 and lysolecithin changes following myocardial infarction", CLINICA CHIMICA ACTA, ELSEVIER BV, AMSTERDAM, NL, vol. 205, no. 3, 14 February 1992 (1992-02-14), pages 223-231, XP025217764, ISSN: 0009-8981, DOI: 10.1016/0009-8981(92)90063-V [retrieved on 1992-02-14]**

## Description

The present invention relates to a new marker liable to be used for the prognostic and the diagnostic of cardiovascular disorders.

Cardiovascular disorders are among the leading causes of morbidity and mortality in developed countries, making the prevention of those afflictions a major concern of public health. One of the steps towards the prevention of those disorders is the detection of individuals at risk. Thus, several biochemical risk markers, such as HDL cholesterol levels, are currently in use for the detection of patients at risk for cardiovascular diseases. However, as of today, numerous cardiovascular disorders can occur in individuals which are not considered at risk relatively to those markers. Therefore, there is a need for new biochemical markers liable to yield a more accurate prediction of cardiovascular events. The sPLA2 plasma level is one of the potential risk markers currently investigated.

Phospholipase A2 (PLA2) enzymes hydrolyze phospholipids at the sn-2 position to generate lysophospholipids and fatty acids (Dennis J Biol Chem. (1994) 269:13057-13060). One of the most extensively studied PLA2 is a low molecular weight (14 kDa) group Ha secretory PLA2 (sPLA2), shown to be expressed in normal arteries and atherosclerotic plaques (Elinder et al. Arterioscler Thromb Vase Biol. (1997) 17:2257-63). Several data suggest a potentially important role for sPLA2 in bath the development and complications of atherosclerosis. The release of arachidonic acid by sPLA2 is a rate limiting event in the generation of oxidation derivatives, including prostaglandins, thromboxanes and leukotrienes. sPLA2 is involved in the oxidative modification of low density lipoprotein (LDL) by lipoxygenase (Mangin et al. Circ Res. (1993) 72:161-6) and in the release of biologically active oxidized phospholipids from both LDL and HDL particles (Leitinger et al. Arterioscler Thromb Vase Biol. (1999) 19:1291-8), suggesting a direct role in atherogenesis.

Thus, several clinical studies have aimed at determining the predictive value of sPLA2 plasma level relative to several cardiovascular disorders. Accordingly, it is considered that the plasma level of sPLA2 is elevated in patients with coronary artery disease and predicts coronary events in stable patients (Kugiyama et al. Circulation. (1999) 100:1280-4). Furthermore, increased plasma level of sPLA2 in stable patients undergoing percutaneous coronary angioplasty also provides independent prognostic information over other classic biological and clinical variables (Liu et al. Eur Heart J. (2003) 24:1824-32). Besides, studies evaluating the prognostic value of sPLA2 in patients with ACS (Acute Coronary Syndrome), limited to a small study of patients with unstable angina, showed that increased plasma levels of sPLA2 predicted recurrent coronary events, mainly revascularization procedures, independently of other risk factors (Kugiyama et al. Am J Cardial. (2000) 86:718-22). Another study measures serum PLA2 activity and lysolecithin in patients following a myocardial infarction by comparison with patients suffering from unstable angina (Langton and Jarnicki (1992) Clin. Chim. Acta 205: 223-231). Finally, Schaefer et al. (J. Clin. Chem. Clin. Chem. (1990) 28: 253-254) investigates whether elevated sPLA activity in intensive care patients, irrespective of the diagnosis or severity of disease, can be used to predict lethality.

However, those studies are much debated, since they have been carried out on too small a number of patients to be considered statistically significant. Indeed, recent studies performed on a larger number of patients indicate that there is no significant correlation between sPLA2 plasma levels and the prediction of mortality or cardiovascular events.

Accordingly, one of the aim of the present invention is to provide a new prognostic and/or diagnostic method of cardiovascular diseases, more reliable than the ones of the prior art.

Another aim of the invention is to provide a new prognostic and/or diagnostic method of cardiovascular diseases liable to be meaningful for apparently healthy people.

## SUMMARY

The present invention thus relates to a method for determining an increased risk of mortality due to a cardiac and/or vascular event or of myocardial infarction in a patient who has been diagnosed as being substantially healthy wherein substantially healthy meant that said patient presents no symptoms of affliction by a disease, in particular with respect to atherosclerosis, cardiac and/or vascular related diseases, comprising:

- determining the secretory Phospholipase A2 (sPLA2) activity in a biological sample of said patient,
- comparing said activity to a predetermined value, wherein the predetermined value corresponds to a sPLA2 activity comprised in the highest two tertiles of the sPLA2 activity range of a given population of substantially healthy individuals, in particular in the higher half, more particularly in the highest tertile of said range,

a higher sPLA2 activity of said patient as compared to said predetermined value being indicative of an increased risk of mortality due to a cardiac and/or vascular event or of myocardial infarction.

In one embodiment, no onset of ischemic symptoms has been diagnosed in the patient.

In one embodiment, the sPLA2 activity of the patient is higher than about 1.8 nmol/ml/min, in particular higher than about 2 nmol/ml/min, more particularly higher than about 2.5 nmol/ml/min, preferably higher than about 2.9 nmol/ml/min, and more preferably higher than 3.3.

In one embodiment, the sPLA2 activity is measured in a biological sample, in particular a serum or a plasma sample, and more particularly a heparinized plasma sample.

The present invention further relates to an in vitro or ex vivo method for the diagnosis of myocardial infarction in a patient who has been diagnosed as being substantially healthy wherein substantially healthy meant that said patient presents no symptoms of affliction by a disease, in particular with respect to atherosclerosis, cardiac and/or vascular related diseases, comprising:

- determining the sPLA2 activity of said patient,
- comparing said activity to a predetermined value, wherein the predetermined value corresponds to a sPLA2 activity comprised in the highest two tertiles of the sPLA2 activity range of a given population of substantially healthy individuals, in particular in the higher half, more particularly in the highest tertile of said range,

a higher sPLA2 activity of said patient as compared to said predetermined value being indicative of myocardial infarction.

In one embodiment, a higher sPLA2 activity of a patient as compared to a predetermined value is indicative of the affliction of said patient with myocardial infarction at the time the determination of the sPLA2 activity of said patient is made, or is indicative of a future affliction of said patient with myocardial infarction, in particular more than 72 hours after the time the determination of the sPLA2 activity of said patient is made.

In one embodiment, the sPLA2 activity of the patient is higher than about 1.8 nmol/ml/min, in particular higher than about 2 nmol/ml/min, more particularly higher than about 2.5 nmol/ml/min, preferably higher than about 2.9 nmol/ml/min, and more preferably higher than about 3.3 nmol/ml/min.

In one embodiment, the sPLA2 activity is measured in a biological sample, in particular a serum or a plasma sample, and more particularly a heparinized plasma sample.

By "increased risk" it is meant that a patient for whom the sPLA2 activity is higher than the predetermined value is more likely to decease or to be afflicted with a cardiac and/or vascular event than an individual for whom the sPLA2 activity is below said predetermined value.

The following events are in particular considered as being cardiac and/or vascular events: myocardial infarction (MI), vascular cerebral accident, hospitalization due to cardiac and/or vascular diseases, and revascularization procedures.

The measure of sPLA2 activity can be performed by a fluorimetric assay according to Radvanyi et al. (1989) Anal Biochem. 177:103-9 as modified by Pernas et al. (Pernas et al. Biochem. Biophys. Res. Commun. (1991) 178:1298-1305). In particular, the following assay is used. The 1-hexadecanoyl-2-(1-pyrenedecanoyl)-sn-glycero-3-phosphomethanol sodium salt (Interchim, Montluçon, France) is used as a substrate for sPLA2. The hydrolysis of this substrate by sPLA2 yields 1-pyrenedecanoic acid, which emits fluorescence at 397 nm. A volume (E) of 0.03 ml of the aliquoted plasmas is mixed with 5 nmol of substrate in presence of a 10 mM Tris-HCL pH 8.7, 0.1% albumin, 10 mM CaCl$_2$ a total volume of 2.5 ml, and fluorescence (F) is measured at 397 nm after one minute. 100% hydrolysis of the substrate is obtained with 0.1 U of bee venom PLA2 (Sigma Chemical Co., France) during one minute, the value of the fluorescence at the end of the one minute reaction (Fmax) thus corresponds to an activity of 2 nmoles/min (Vmax). The activity (A) of the sample (expressed in nmol/ml/min) is given by the following formula:

$$A = \frac{V \max . F}{E . F \max}$$

The samples are diluted when substrate hydrolysis is above 50%. The hydrolysis of substrate in the absence of plasma is used as negative control and deduced from PLA2 activity. Ali samples are tested in duplicate. The minimum detectable activity and detection limit is 0.10 nmole/min/ml and the intra and interassay coefficient of variation is lower than 10%.

All the numerical values given herein for plasma sPLA2 activity are measured according to the above defined assay.

The expression "predetermined value" designates a threshold value above which a plasma sPLA2 activity is deemed to be significant of a present or future affliction of a patient with a cardiac and/or vascular related event or pathology.

The expression "higher sPLA2 activity" means that to be indicative of an increased risk, the value of the sPLA2 activity of the patient must be greater than the predetermined value.

According to the above defined method, the patient has been diagnosed as being substantially healthy, in particular with respect to atherosclerosis, cardiac and/or vascular related diseases.

By "substantially healthy" is meant that the patient presents no symptom of affliction by a disease, in particular by an atherosclerosis, cardiac and/or vascular related disease. Such diseases comprise in particular: coronary artery diseases (CAD), carotid atherosclerosis, aortic atherosclerosis, iliac or femoral atherosclerosis, vascular aneurysm, vascular calcification, hypertension, heart failure, and diabetes.

According to another particular embodiment of the above defined method, the patient has been diagnosed as presenting one of the following coronary disorders:

- asymptomatic coronary artery disease with silent ischemia or without ischemia,
- chronic ischemic disorders without myocardial necrosis, such as stable or effort angina pectoris,
- acute ischemic disorders without myocardial necrosis, such as unstable angina pectoris,
- ischemic disorders with myocardial necrosis, such as ST segment elevation myocardial infarction or non-ST segment elevation myocardial infarction.

Tissue ischemia is often defined in relative terms and occurs when the needs in oxygen exceed the delivery of oxygen to tissues. There is an imbalance between tissue (myocardial for example) oxygen demands and supply. This condition of oxygen deprivation may be accompanied by inadequate removal of metabolites consequent to reduced perfusion. Myocardial ischemia can be diagnosed clinically (chest pain for example), biologically (increase in myeloperoxidase activity for example), metabolically, using scintigraphy, by analyzing regional wall motion disorders or by use of an electrocardiogram (typical modifications of the ST segment, upper or lower ST segment deviation, typical changes in T wave such as T wave inversion or steep symmetric or high amplitude positive T waves). Silent ischemia is typically diagnosed using scintigraphy or a 24h electrocardiogram recording.

Stable and effort angina is typically manifested by a chest pain during exercise and slowly recovers at rest. It usually reflects tissue ischemia during exercise.

Unstable angina is either a recent increase in the frequency and/or severity of stable angina, a first episode of angina, or an angina at rest.

Myocardial necrosis is typically diagnosed by an increase in myocardial enzymes (for example troponin I, troponin T, CPK) in the circulating blood.

According to a more particular embodiment of the above defined method, the patient has a medical suspicion of the following coronary disorders:

- asymptomatic coronary artery disease with silent ischemia or without ischemia,
- chronic ischemic disorders without myocardial necrosis, such as stable or effort angina pectoris,
- acute ischemic disorders without myocardial necrosis, such as unstable angina pectoris,
- ischemic disorders with myocardial necrosis, such as ST segment elevation myocardial infarction or non-ST segment elevation myocardial infarction.

According to another particular embodiment of the above defined method, no onset of ischemic symptoms has been diagnosed in the patient.

Myocardial ischemia can be diagnosed, clinically (chest pain for example), biologically (increase in myeloperoxidase activity for example), metabolically, using scintigraphy, by analyzing regional wall motion disorders or by use of an electrocardiogram (typical modifications of the ST segment, upper or lower ST segment deviation, typical changes in T wave such as T wave inversion or steep symmetric or high amplitude positive T waves).

According to yet another particular embodiment of the above defined method, an onset of ischemic symptoms has been diagnosed in the patient.

In a preferred embodiment of the above defined method, the predetermined value corresponds to a sPLA2 activity comprised in the highest two tertiles of the sPLA2 activity range of a substantially healthy individual, in particular in the higher half, more particularly in the highest tertile of said range.

In another preferred embodiment of the above defined method, the predetermined value corresponds to a sPLA2 activity comprised in the sPLA2 activity range of the two thirds, in particular the half, more particularly the third, of the individuals having the highest sPLA2 activity relative to all the individuals constituting a given population of substantially healthy individuals.

As intended above, a given population of substantially healthy individuals is divided in three parts (or tertiles), each containing the same number of individuals, one tertile corresponding to the part of the given population with the highest sPLA2 activities, a second tertile corresponding to the part of the given population with the lowest sPLA2 activities, and the third tertile corresponding to the rest of the population.

In a particularly preferred embodiment of the above defined method, the sPLA2 activity of the patient is higher than about 1.8 nmol/ml/min, in particular higher than about 2 nmol/ml/min, more particularly higher than about 2.5 nmol/ml/min, preferably higher than about 2.9 nmol/ml/min, and more preferably higher than 3.3.

In a preferred embodiment of the above defined method, the sPLA2 activity of the patient is lower than about 7 nmol/ml/min.

In another preferred embodiment of the above defined method, the sPLA2 activity of the patient is higher than about 1.8 nmol/ml/min and lower than 7 nmol/ml/min, in particular higher than about 2 nmol/ml/min and lower than 7 nmol/ml/min, more particularly higher than about 2.5 nmol/ml/min and lower than 7 nmol/ml/min, preferably higher than about 2.9 nmol/ml/min and lower than 7 nmol/ml/min, and more preferably higher than 3.3 and lower than 7 nmol/ml/min.

In a further preferred embodiment of the above defined method, the sPLA2 activity of the patient is higher than about 3.3 nmol/ml/min and lower than 7 nmol/ml/min.

The sPLA2 activity is measured according to the above mentioned assay method, in particular the substrate used is the sodium salt of 1-hexadecanoyl-2-(1-pyrenedecanoyl)-sn- glycero-3-phosphomethanol and the standardizing PLA2 is the bee venom PLA2.

In another particularly preferred embodiment of the above defined method, the sPLA2 activity is measured in a biological sample, in particular a serum or a plasma sample, and more particularly a heparinized plasma sample.

By heparinized plasma sample, it is meant that the plasma collecting tubes have been heparinized prior to plasma addition, in particular with unfractionated heparin.

The present application also relates to an in vitro or ex vivo method for the diagnosis of cardiac and/or vascular diseases in a patient, comprising:

- determining the sPLA2 activity of said patient,
- comparing said activity to a predetermined value,

a higher sPLA2 activity of said patient as compared to said predetermined value being indicative of a cardiac and/or vascular disease.

By cardiac and/or vascular diseases are intended afflictions such as: coronary artery disease (CAD), hypertension, atherosclerosis, iliac or femoral atherosclerosis, vascular aneurysm, vascular calcification, hypertension, heart failure, and diabetes.

In a particular embodiment of the above defined in vitro or ex vivo method, a higher sPLA2 activity of a patient as compared to a predetermined value is indicative of the affliction of said patient with a cardiac and/or vascular disease at the time the determination of the sPLA2 activity of said patient is made, or is indicative of a future affliction of said patient with a cardiac and/or vascular disease, in particular more than 72 hours after the time the determination of the sPLA2 activity of said patient is made.

By "future affliction" is meant an affliction the onset of which occurs after the moment when the determination of the sPLA2 activity of said patient has been made.

In another particular embodiment of the above defined *in vitro* or *ex vivo* method, the sPLA2 activity of the patient is higher than about 1.8 nmol/ml/min, in particular higher than about 2 nmol/ml/min, more particularly higher than about 2.5 nmol/ml/min, preferably higher than about 2.9 nmol/ml/min, and more preferably higher than about 3.3 nmol/ml/min.

In a preferred embodiment of the above defined *in vitro* or *ex vivo* method, the sPLA2 activity of the patient is lower than about 7 nmol/ml/min.

In another preferred embodiment of the above defined *in vitro* or *ex vivo* method, the sPLA2 activity of the patient is higher than about 1.8 nmol/ml/min and lower than 7 nmol/ml/min, in particular higher than about 2 nmol/ml/min and lower than 7 nmol/ml/min, more particularly higher than about 2.5 nmol/ml/min and lower than 7 nmol/ml/min, preferably higher than about 2.9 nmol/ml/min and lower than 7 nmol/ml/min, and more preferably higher than 3.3 and lower than 7 nmol/ml/min.

In another particular embodiment of the above defined in vitro or ex vivo method, the sPLA2 activity is measured in a biological sample, in particular a serum or a plasma sample, and more particularly a heparinized plasma sample.

## DESCRIPTION OF THE FIGURES

### Figure 1

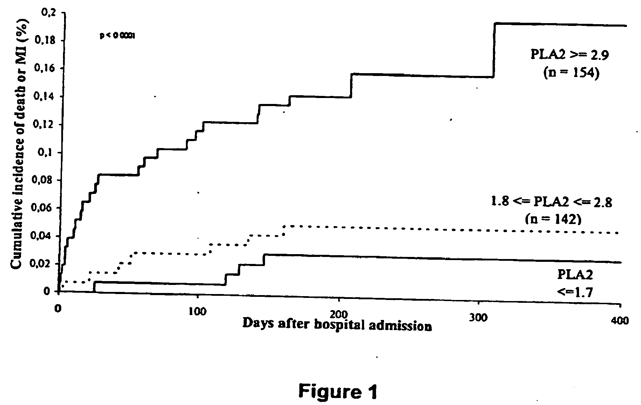

Figure 1 represents the cumulative incidence of death and myocardial infarction (MI) (vertical axis, in%) versus the days after hospital admission (horizontal axis) of patient with a sPLA2 plasma activity lower than 1.7 nmol/min/ml, between 1.8 and 2.8 nmol/min/ml, and above 2.9 nmol/min/ml.

### Figure 2

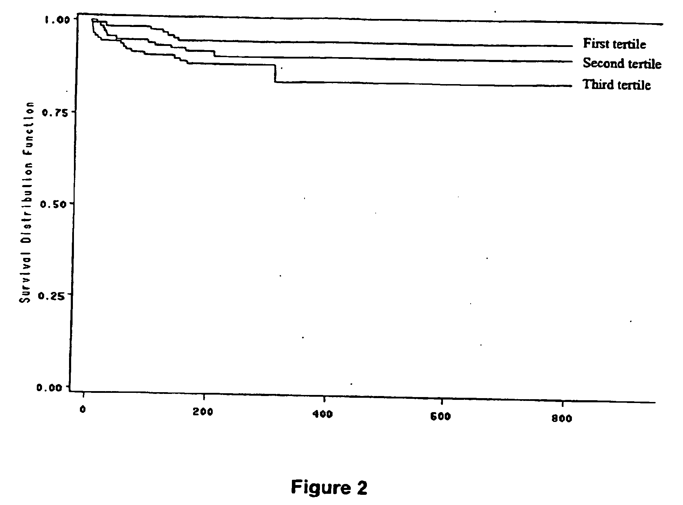

Figure 2 represents the proportion of patients unaffected by death and myocardial infarction (MI) (vertical axis) versus the days after their hospital admission (horizontal axis), the patients having respectively a sPLA2 plasma level in the first, the second or the third tertile of the overall repartition of sPLA2 plasma levels for these patients.

## EXAMPLES

## Example 1

Methods

**Study population**

Full details of the GRACE methodology have been published (Steg et al. (2004) Circulation 109:494-9). Patients included in this substudy (from September 28th, 2000 to October 24th, 2002) were recruited in 3 centers in France and one center in Scotland. Inclusion criteria for the present study were age $\geq$ 18 years, a preemptive diagnosis of ACS (Acute Coronary Syndrome) and at least one of the following criteria: dynamic ECG changes consistent with ACS, serial increases in serum biochemical markers of cardiac necrosis, and/or documentation of coronary artery disease. Exclusion criteria were recent myocardial infarction with persistent elevation of serum biochemical markers of cardiac necrosis, Killip class IV, trauma, surgery, significant co-morbidity, life expectancy of less than 6 months, a diagnosis of cancer, HIV positivity, nocturnal paroxysmal hemoglobinuria, hemolytic uremie syndrome, thrombotic thrombocytopenic purpura, auto-immune thrombocytopenia, heparin- induced thrombocytopenia, anti-phospholipid syndrome, systemic lupus erythematosus and Crohn's disease. At 6 months after hospital discharge, patients were followed up by telephone, clinic visits, or calls to the primary care physician.

Standardized definitions of ail patient-related variables and clinical diagnoses were used as well as hospital complications and outcomes. Ail cases were assigned to one of the following categories: STEMI (ST-segment Elevation Myocardial Infarction), NSTEMI (Non-ST-segment Elevation Myocardial Infarction) or unstable angina. These definitions take into account clinical presentation, BCG findings, and the results of serum biochemical markers of necrosis. Unstable angina was defined as ACS with normal biochemical markers of necrosis (particularly troponin I). Patients were categorized at the time of hospital admission according to the classification of Killip and Kimbail for signs of heart failure.

**Blood sampling and biochemical analyses**

Blood samples were collected in heparin tubes at the time of enrollment (the first working morning following admission) and immediately centrifuged at 3,000 rpm at 4°C for 10 minutes. The resulting plasma were aliquoted, frozen, and shipped on dry ice to Xavier Bichat-Claude Bernard Hospital (Paris, France) where they were maintained at -70°C until analyzed.

C-reactive protein (CRP) was measured by a high-sensitivity test performed on a Behring BN II analyzer, (Behring Diagnostics) and level of cardiac tropon.in I (cTnI) was quantified by use of an automated system (RXL HM analyzer, Dade Behring).

High-sensitivity interleukin-18 (IL-18) was measured using a human ELISA kit (MBL Co , Japan) with a detection limit of 12.5 pg/ml.

Levels of immunoreactive sPLA2 in plasma were measured by an immunometric assay based on a double-antibody "sandwich" technique, using a monoclonal antibody specific for type IIA secreted PLA2 (Cayman Chemical Company, USA). This antibody has no cross-reactivity with type I, IV, or type V sPLA2 (Cayman Chemical Company, USA). The minimum detectable concentration was 15.6 pg/ml and the intra and interassay coefficient ofvariation (CV) was < 10%.

Plasma sPLA2 activity was measured by a selective fluorimetric assay of Radvanyi et al. (1989) Anal. Biochem. 177:103-9 as modified by Pernas et al. (1991) Biochem. Biophys. Res. Commun. 178:1298-1305. Briefly, the 1-hexadecanoyl-2-(1-pyrenedecanoyl)-sn-glycero-3-phosphomethanol sodium salt (Molecular Probe) was used as a substrate for sPLA2. The hydrolysis of this substrate by sPLA2 yields 1-pyrenedecanoic acid, which emits fluorescence at 397 nm. A volume of 0.03 ml (E) of the aliquoted plasmas was mixed with 5 nmol of substrate in presence of a 10 mM Tris-HCL pH 8.7, 0.1 % albumin, 10 mM CaCl2 in a total volume of 2.5 ml, and fluorescence (F) was measured at 397 nm after one minute. 100% hydrolysis of the substrate is obtained with 0.1 U of bee venom PLA2 (Sigma Chemical Co., France) during one minute, the value of the fluorescence at the end of the one minute reaction (Fmax) thus corresponds to an activity of 2 nmoles/min (Vmax). The activity (A) of the sample (expressed in nmol/min/ml) is given by the following formula:

$$A = \frac{V max . F}{E . F max}$$

The samples were diluted when substrate hydrolysis was above 50%. The hydrolysis of substrate in the absence of plasma was used as negative control and deduced from PLA2 activity. Ali samples were tested in duplicate. The minimum detectable activity and detection limit was 0.10 nmole/min/ml and the intra and interassay CV was < 10%.

**Statistical analysis**

Potential associations between plasma sPLA2 activity and clinical parameters were tested in univariate analysis using Student's t tests or ANOVA for categorical variables and Pearson correlation for age. Univariate predictors with p <= 0.05 were included in a stepwise multivariate linear regression analysis.

Correlations between plasma sPLA2 activity and biological variables were assessed by Pearson correlation coefficients. Comparisons between groups of patients according to tertiles of sPLA2 activity were performed by ANOVA for continuous variables and chi-square or Fisher exact test for categorical variables. Survival curves were derived from Kaplan-Meier estimates. Overall survival was defined as the time interval between the date of hospital admission and the date of death from all causes. Patients alive at last follow-up were censored. The MI (Myocardial Infarction) free survival was defined as the time interval between the date of hospital admission and the date of myocardial infarction recurrence. Death and patients alive without recurrence of MI at last follow-up were censored. For the combined endpoint, survival was defined as the time interval between the date of hospital admission and the date of first event. Patients alive without recurrence of MI at last follow-up were censored. Comparisons of the survival distributions were made by log rank tests.

Independent predictors of death, MI and death or MI were identified using a Cox's proportional hazards regression model. Variables included in the model were univariate predictors with p<0.05. Ali analyses were performed with the SAS software version 8.2 (SAS Institute, Cary, NC).

**Results**

The study population consisted of 458 patients with ACS, 38.5% with STEMI, 52.5% with NSTEMI and 49% with unstable angina. The median follow-up after admission was 6.5 months (25th percentile: 6 months, 75th percentile: 7.5 months). Mean time ($\pm$SD) from onset of clinical symptoms to blood sampling was 30.05$\pm$21.3 hours. This time interval was not correlated with plasma levels of sPLA2 (P=0.45). Plasma sPLA2 activity ranged from $\leq$ 0.1 to 16.9 nmol/min/ml with a mean of 2.63$\pm$1.69 nmol/min/ml. One year cumulative incidence of death or MI: 9.63% (S.E: 0.02).

**Association of plasma sPLA2 activity with baseline clinical and biological variables**

In univariate analyses, clinical variables associated with higher sPLA2 activity were patient recruitment in Scotland (versus recruitment in France), male sex, age, a history of diabetes, hypertension, and a clinical presentation with STEMI or Killip class >1. Treatment by unfractionated heparin within first 24 hours was also associated with higher sPLA2 activity. A history of angina, myocardial infarction, coronary angiography or angioplasty, a history of hyperlipidemia or treatment by LMW (Low Molecular Weight) heparin were associated with lower sPLA2 levels. In multivariate analyses, recruitment center, age, diagnosis of STEMI at admission, presentation with Killip class >1 and treatment by unfractionated heparin were significant predictors of high sPLA2 activity **(Table 1).** A history of hyperlipidemia was significantly associated with lower sPLA2 activity **(Table 1).**

When patient population was studied according to tertiles of sPLA2 activity **(Table-2),** higher age, a history of heart failure, Killip class >1 at admission, and higher Tni and CRP values were more frequently associated with higher sPLA2 activity, whereas a history of coronary angioplasty, hyperlipidemia or higher cholesterollevels measured during the first 24 hours following admission were more frequent in the lower tertiles of sPLA2 **(Table 2).**

Plasma sPLA2 activity correlated modestly with CRP levels (r=0.47, P < 0.0001). Although statistically significant, the associations between sPLA2 activity and TnI levels (r=0.12, P=0.01), total cholesterol levels (r=-0.17, P=0.009) and LDL cholesterol (r=-0.18, P=0.007) were weak. There was no correlation between sPLA2 activity and IL-18 levels (i=0.02, P=0.74). Besides, the correlation between sPLA2 plasma activity and sPLA2 plasma antigen level is unexpectedly low (i=0.66, P<0.0001); as a consequence, results obtained for sPLA2 activity cannot be inferred from sPLA2 plasma level; this unexpected finding could be explained by the presence of a substantial amount of non or slightly active sPLA2 enzymes in plasma.

**Association of baseline plasma sPLA2 activity with clinical outcomes**

The mean baseline sPLA2 activity was higher among patients who died or had new or recurrent MI than among those who were alive and free of MI at 6 months of follow-up (3.31$\pm$1.22 vs. 2.57$\pm$1.72, respectively, P=0.02). No association was seen between clinical outcomes and either CRP or IL-18 levels.

The rate of death and new or recurrent MI increased according to increasing tertiles of sPLA2 activity. Patients in the highest tertile of sPLA2 had a relative risk of 4.30 (95 percent confidence interval, 2.1 to 8.7) for death and MI (P<0.0001) (Figure 1). This association remained significant in subgroups of patients with STEMI (RR=6.9; 1.5-31.8), patients with NSTEMI or unstable angina (RR=3.8; 1.7-8.7), patients with Killip class <1 (RR=3.8; 1.4-10.4), patients with Killip class >1 (RR=2.8; 1.0-7.7), patients with CRP<10 mg/l (4.7; 1.8-12.1) and patients with CRP levels>10 mg/l (4.0; 1.3-12.2).

Adjustment for potential confounders in a Cox regression made1, including age, a history of hypertension, diabetes, myocardial infarction, heart failure, coronary angiography or angioplasty, Killip class, ST-segment deviation, coronary revascularization (angioplasty or coronary artery bypass surgery), and creatinine did not alter the strong association between high base-line levels of sPLA2 activity and increased risk of major coronary events at follow-up (**Table 3**).

The adjusted relative risk for death, non-fatal MI or the combined endpoint of death and MI in the third tertile of sPLA2 were 2.61 (95 percent confidence interval, 1.01 to 6.39) (P=0.036), 6.1 (95 percent confidence interval, 1.91 to 19.21) (P=0.002) and 3.3 (95 percent confidence interval, 1.56 to 6.85) (P=0.002), respectively.

Furthermore, the adjusted relative risk for the combined endpoint of death and MI for a plasma sPLA2 activity higher than 1.8 was 2.44 (95 percent confidence interval, 0.70 to 8.48), 2.19 (95 percent confidence interval, 0.73 to 6.58) for a plasma sPLA2 activity higher than 2, 2.4 (95 percent confidence interval, 1.02 to 5.65) for a plasma sPLA2 activity higher than 2.5, 3.08 (95 percent confidence interval, 1.37 to 6.91) for a plasma sPLA2 activity higher than 2.9 and 2.26 (95 percent confidence interval, 1.11 to 4.66) for a sPLA2 plasma activity higher than 3.3 (N.B. sPLA2 plasma activity is expressed in nmol/minlml).

No significant association was found between sPLA2 antigen level and the composite endpoint of death or MI (relative risk, 1.44, P=0.3). Finally, it was verified that the GRACE score, previously shown to be highly predictive of event recurrence (Eagle et al. (2004) Jama. 291:2727-33), significantly predicted adverse outcomes in the present study (relative risk for each one point increase in score, 1.03; 95 percent confidence interval, 1.02 to 1.04, p<0.0001). Interestingly, in a Cox regression model including GRACE score, the adjusted relative risk for the combined endpoint of death or MI in the third tertile of sPLA2 activity was 2.78 (95 percent confidence interval, 1.30 to 5.93) (P=0.006). sPLA2 antigen level added no significant prognostic information to the GRACE score (P=0.3).

**Comparison with other markers**

Inflammatory and thrombotic processes are major determinants of atherosclerotic plaque complications leading to acute coronary syndromes (ACS) and sudden death. In addition to pathological studies showing an important association between plaque inflammation and plaque rupture in humans and experimental data showing a critical role of the immuno-inflammatory response both in plaque development and composition, the last decade has witnessed an increasing interest in the study of the role of systemic inflammatory markers and their relation to the severe clinical complications of atherosclerosis. Several circulating inflammatory markers, including CRP, IL-6, IL-1 receptor antagonist (IL-Ira), vascular cell adhesion molecule (VCAM)-1, and more recently myeloperoxidase, have been shown to be elevated in patients with ACS and to be associated with adverse clinical outcomes at follow-up. In this study, the Inventors have shown that plasma sPLA2 activity, in contrast to CRP or IL-18, is a major independent predictor of death and new or recurrent MI in patients with ACS. Since the landmark study by Liuzzo et al. (Liuzzo et al. (1994) N Engl J Med.; 331:417-24), numerous studies have addressed the prognostic value of CRP in patients with ACS. Higher CRP levels were associated with increased risk at follow-up in several randomized trials of patients with unstable angina or NSTEMI, including TIMI Iia (Morrow et al. (1998) J Am Coll Cardiol.; 31:1460-5), CAPTURE (Heeschen et al. (2000) J Am Coll Cardiol.; 35:1535-42), FRISC (Lindahl et al. (2000) N Engl J Med.; 343: 1139-47) and GUSTO-IV (James et al. (2003) Circulation.; 108:275-81). CRP levels in ACS patients assigned to early invasive revascularization procedures also predict adverse outcomes (Mueller et al. (2002) Circulation.; 105:1412-5). However, the independent predictive value of CRP in patients with STEMI or patients with ACS, recruited outside randomized clinical trials, is debatable (Zairis et al. (2002) Am Heart J.; 144:782-9; Bennermo et al. (2003) J Intern Med.; 254:244-50; Benamer et al. (1998) Am J Cardiol.; 82:845-50). In the present observational substudy from GRACE, CRP measured a few hours after admission for ACS (mostly STEMI and NSTEMI) was not associated with adverse outcomes, suggesting that in every day practice, a single early measurement of CRP in patients admitted for ACS may not add significant predictive information over clinical variables.

On the basis of previous experimental data by the Inventors and others showing a potent pro-atherogenic role for endogenous IL-18 (Mallat et al. (2001) Circ Res.; 89:E41-5; Whitman et al. (2002) Circ Res.; 90:E34-8), plasma IL-18 levels have been measured in patients with a history of coronary artery disease and in healthy middle-aged men and was found to be an independent predictor of coronary events (Blankenberg et al. (2002) Circulation.; 106:24-30; Blankenberg et al. (2003) Circulation.; 108:2453-9). In the present study, no association between·IL-18 levels, measured at admission, and adverse outcomes at follow-up in patients with ACS was found. These results taken together suggest that the prognostic value of IL-18 measurement varies according to disease activity at the time of sampling. This could be related to multiple factors, including untested confounding variables, a differential role of 11-18 in different clinical settings or to differences in the production of IL-18 binding protein, the endogenous inhibitor of IL-18. These considerations should be taken into account in future studies evaluating the prognostic implications of IL-18 in patients with ACS.

sPLA2 plasma antigen level

The most important and novel finding in this contemporary study is the demonstration that a single determination of sPLA2 activity, obtained during the 2 days, with a mean of 30 hours, after the onset of ischemic symptoms, provides powerful prognostic information in patients with ACS. The association between sPLA2 activity and the risk of death and myocardial infarction was independent of the other known predictors of major adverse outcomes in patients with ACS, including the presence or absence of a history of myocardial infarction and the presence or absence of clinical signs of heart failure at admission (Steg et al. (2004) Circulation.; 109:494-9). Interestingly, this finding of a strong predictive value of sPLA2 activity was obtained despite the heterogeneity of the population under study regarding clinical presentation, pathophysiology and the risk associated with each type of acute coronary syndromes and the high rate of in-hospital revascularization, consistent with the current everyday practice. This suggests that activation of sPLA2 may be a critical and common mechanism among patients at risk for death and myocardial infarction after acute coronary syndromes.

A previous small study has demonstrated that in patients with unstable angina, a higher plasma level of sPLA2 is associated with a higher probability of developing clinical coronary events, mainly coronary revascularizations and readmissions, during a follow-up period of 2 years (Kugiyama et al. (2000) Am J Cardiol.; 86:718-22). This previous study focused on a limited number of homogeneous patients and included only 52 patients with unstable angina who had no increase in biochemical markers of necrosis. In addition, no direct measurement of sPLA2 activity was performed.

In comparison, the present study has shown that no significant association was found between sPLA2 antigen level and the composite endpoint of death and MI (relative risk, 1.44, P=0.3) as well as for MI alone (relative risk 0.5, P=0.7) (Figure 2). Besides, the present study is extended to patients with severe ACS, and shows that sPLA2 activity is a strong and independent predictor of death and myocardial infarction in patients with STEMI and patients with NSTEMI.

**Benefits of using sPLA2 activity as a risk marker**

The importance of sPLA2 activity assessment in comparison with other previously tested inflammatory markers in acute coronary syndromes is of several orders.

Unlike traditional cardiac biomarkers used to predict adverse outcome in patients with ACS, sPLA2 activity has been shown to act at multiple critical pathways involved in atherogenesis, from lipid oxidation to modulation of vascular and inflammatory cell activation and apoptosis. sPLA2 is expressed in various tissues and could be one of the acute phase reactants (Pruzanski and Vadas (1991) Immunol Today.; 12:143-6; Murakami et al. (1995) J Lipid Mediat Cell Signal.; 12:119-30). Interestingly, sPLA2 is already expressed in the normal arterial wall (Hurt-Camejo et al. (1997) Arterioscler Thromb Vase Biol.; 17:300-9; Elinder et al. (1997) Arterioscler Thromb Vase Biol.; 17:2257-63) and its expression is readily upregulated by inflammatory stimuli (Pruzanski and Vadas (1991) Immunol Today.; 12:143-6; Murakami et al. (1995) J Lipid Mediat Cell Signal.; 12:119-30; Nakano and Arita (1990) FEBS Lett.; 273:23-6, Murakami et al. (1993) J Biol Chem.; 268:839-44, Kuwata et al. (1998) J Biol Chem.; 273:1733-40), suggesting a potential role for sPLA2 beginning in the early phases of the response of the vessel to injury. In contrast to a number of other inflammatory markers, sPLA2 has been shown to be a patent pro-atherogenic enzyme in an animal model of atherosclerosis, being able to induce the formation of fatty streaks even in the absence of elevated cholesterol levels (Ivandic et al. (1999) Arterioscler Thromb Vase Biol.; 19:1284-90), suggesting a direct involvement of sPLA2-mediated pathways in atherosclerosis.

sPLA2 stimulates the oxidation of native LDL (Mangin et al. (1993) Circ Res.; 72:161-6) and is involved in the release of polyunsaturated fatty acids, leading to the production of biologically active phospholipids derived from 1-palmitoyl-2-arachidonoyl-sn-glycero-3phosphorylcholine (PAPC) (Leitinger et al. (1999) Arterioscler Thromb Vase Biol.;19:1291-8). These oxidation products play important roles in platelet, monocyte and endothelial activation and in monocyte-endothelial interactions, ail processes known to be critical steps in atherogenesis. Lipid mediators produced through sPLA2, including prostaglandins, thromboxanes and leukotrienes have shown significant pro-inflammatory, pro-trombogenic and pro-atherogenic effects. Lysophospholipids, including lysophosphatidycholine and lysophosphatidic acid, released from the cell membrane of platelets and other cells in the form of microvesicles following sPLA2 activation (Fourcade et al. (1995) Cell.; 80:919-927), can influence cellular functions (Swarthout and Walling (2000) Cell Mol Life Sci.; 57:1978-85; Kume et al. (1992) J Clin Invest.; 90:1138-44) and lead to the generation of PAF, a patent cell activator and a potentially pro-atherogenic factor.

sPLA2 greatly potentiates the activation of T lymphocytes leading to enhanced proliferation, suggesting that phospholipase A2 secreted into inflammatory sites, including the atherosclerotic plaque (Menschikowski et al. (1995) Atherosclerosis.; 118:173-81; Hurt-Camejo et al. (1997) Artérioscler Thromb Vase Biol.; 17:300-9; Elinder et al. (1997) Arterioscler Thromb Vase Biol.; 17:2257-63), plays a role in the propagation of cellular responses. Of major importance, sPLA2 is virtually inactive on phospholipids from intact cells, and several studies suggest that only those membranes where the transverse distribution of phospholipids has been disturbed offer a convenient surface able to interact with the enzyme (Fourcade et al. (1998) Adv Enzyme Regul.; 38:99-107).

It has previously been shown by the Inventors that increased levels of intra-plaque and circulating shed-membrane microparticles presenting an altered phospholipid distribution with phosphatidylserine exposure at the outer leaflet of the membrane in patients with acute coronary syndromes in comparison with stable and non-coronary patients (Mallat et al. (1999) Circulation.; 99:348-353; Mallat et al. (2000) Circulation.; 101:841-3). These microparticles may constitute an important substrate for sPLA2 both at the site of plaque complication and in the circulating blood. Taken together, these data suggest that sPLA2 activity, in the arterial wall and the circulating blood, may play a critical role in inflammatory pathways leading to acute coronary syndromes, and may explain, at least in part, the 6-fold increase in new or recurrent myocardial infarction in patients with ACS.

For sPLA2 activity to be useful in the clinical assessment of patients with ACS, it must help clinicians in therapeutic decision making. Patients with low levels of sPLA2 activity appear to have a particularly low risk of major adverse events at follow-up, suggesting that, in these patients, an aggressive or invasive therapeutic strategy could be avoided, leading to reduction in costs and risk of unnecessary therapy. On the other hand, patients with elevated levels of sPLA2 activity are at increased risk of MI and death and may benefit from a more aggressive medical and/or revascularization therapy. These issues should be addressed specifically in future studies.

In conclusion, the level of sPLA2 activity, measured a few hours after the onset of ischemic symptoms in patients with acute coronary syndromes, is a strong predictor of death and MI at 6 months of follow-up. Importantly, the risk associated with elevated sPLA2 activity is statistically independent of other clinical and biological markers associated with increased risk. An additional strength of these findings is that they were obtained in an observational contemporary study conducted in the "real world" setting. Thus, measurement of sPLA2 activity will provide a valuable additional information for use in risk stratification in the future.

## Example 2

Following the methodology outlined in Example 1, a study on a very similar study sample was carried out. The results obtained are identical to the conclusions of **Example 1.**

The study sample consisted of 446 patients with ACS, 38.5% with STEMI, 52.5% with NSTEMI and 9% with unstable angina. The median follow-up after admission was 6.5 months (25th percentile: 6 months; 75th percentile: 7.5 months). The rate of in-hospital revascularization (PCI or CABG) was high: 48.3%. One year cumulative incidence of death or MI was: 9.63% (S.E: 0.02). Plasma sPLA2 activity ranged from $\leq 0.1$ to 16.9 nmole/min/ml with a mean of $2.63\pm1.69$ nmole/min/ml. The time interval from onset of clinical symptoms to blood sampling was not associated with plasma levels of sPLA2 activity (P=0.45).

### Association of plasma sPLA2 activity with base-line clinical and biological variables

Sample characteristics are shown in **Table 4** and clinical variables associated with sPLA2 activity are shown in **Table 5.** In multivariable analyses, significant predictors of high sPLA2 activity were recruitment center, age, diagnosis of STEMI at admission , presentation with Killip class >1 and treatment by unfractionated heparin **(Table 5).** A history of hyperlipidemia was significantly associated with lower sPLA2 activity **(Table 5).** Plasma sPLA2 activity showed moderate association with CRP (i=0.35, P<0.0001). Although statistically significant, the associations between sPLA2 activity and TnI (r=0.23, P<0.0001), total cholesterol (r= -0.24, P<0.0001) and LDL cholesterol (r=-0.24, P=0.0003) were weak. There was no association between sPLA2 activity and IL-18 levels (r=0.03, P=0.53). sPLA2 activity showed moderate association with sPLA2 antigen levels (r=0.37, P<0.0001). This was not unexpected since sPLA2 activity is that of the various sPLA2 subtypes, whereas sPLA2 antigen levels only reflect the levels of the type HA sPLA2.

### Association of base-line plasma sPLA2 activity with clinical outcomes

There were 35 major adverse events (20 deaths and 15 MI). No significant association was seen between clinical outcomes and either CRP or IL-18 levels **(Table 6).** The rate of death and new or recurrent MI increased according to increasing tertiles of sPLA2 activity. Patients in the highest tertile of sPLA2 activity had a hazard ratio of 4.30 (95 percent confidence interval, 2.1 to 8.7) for death or MI (P<0.0001) (compared to the other 2 tertiles) **(Table 6).** Adjustment for potential confounders in a stepwise Cox regression model, including age, a history of hypertension, myocardial infarction, heart failure, coronary artery disease, coronary artery bypass surgery, Killip class>1, and creatinine level >1.1 did not alter the strong association between high base-line levels of sPLA2 activity and increased risk of major coronary events at follow-up **(Table 6).** The adjusted hazard ratio for the combined endpoint of death or MI in the third tertile of sPLA2 activity compared to the first and second tertiles was 3.08 (95 percent confidence interval, 1.37 to 6.91) (P=0.006) **(Table 7).**

No significant association was found between sPLA2 antigen level and the composite endpoint of death or MI (adjusted hazard ratio, 1.44, P=0.3). Finally, it was verified that the GRACE score, previously shown to be highly predictive of event recurrence, significantly predicted adverse outcomes (MI/death) in the present study (hazard ratio for each one point increase in score, 1.03; 95 percent confidence interval, 1.02 to 1.04, P<0.0001). Interestingly, sPLA2 activity remained an independent predictor for recurrent events after adjusting for the GRACE score for recurrent events in a Cox regression model. The adjusted hazard ratio for the combined endpoint of death or MI in the third tertile of sPLA2 activity was 2.78 (95 percent confidence interval, 1.30 to 5.93) (P=0.006).

sPLA2 activity was higher in patients recruited in Scotland than in patients recruited in France **(Table 5).** This may be related, at least in part, to the higher age of patients recruited in Scotland (P<0.0001 in comparison with France), and the higher percentage of Scottish patients with a Killip class>1 at admission (34.52% vs only 12.02% in the French sample of patients) (P<0.0001).

### Example 3

Preliminary results of a study conducted on a distinct population confirm the results obtained in **Example 1,** for the use of sPLA2 activity as a risk marker for cardiac and vascular events.

1105 individuals are included in the present study. These individuals were selected as having developed a coronary disease (lethal or not) until November 2003 (mean follow-up duration of 6 years). 2209 matched controls for sex, age and date of inclusion were also part of the study.

The individuals were selected among the participant of the EPIC-Norfolk study. The latter is a prospective study of a population including 25 663 men and women aged 45 to 79, living in Norfolk (United Kingdom) and having filled a questionnaire and undergone a clinical check-up at the time of the inclusion (between 1993 and 1997) (Day N, et al. EPIC-Norfolk: study design and characteristics of the cohort. European Prospective Investigation of Cancer. Br J Cancer 1999; 80 Supp11:95-103). By way of example, the characteristics of 1400 control individuals and 700 patients included in the EPIC-Norfolk study are presented in **Table 8.** Each individual can be identified from the UK Office of National Statistics for death certificates, and the living status is precisely known for the whole cohort. Patients admitted to a hospital are identified thanks to their unique National Health Service number in connection with East Norfolk Health Authority database. Patients have been identified as being afflicted with a coronary disease during follow-up if they have been hospitalized with a diagnostic of coronary disease and/or died from the consequences of a coronary disease. Coronary disease has been defined according to codes 410 to 414 of the International Classification of Diseases, 9th edition. These codes include the whole clinical panel of coronary diseases from stable angor to infarction.

A venous blood sample was taken at the time of inclusion in citrated dry tubes. Blood samples (plasma and serum) were kept at -80°C in the Department of Clinical Biochemistry, University of Cambridge, UK.

The samples of the 1105 case individuals and of the 2209 controls were transported in dry ice and were kept at -80°C in the Biochimie B department of Bichat Hospital, Paris sPLA2 activity is measured according to the method described in Example 1.

Cardiovascular risk factor distribution according to quartiles of sPLA2 activity is determined to analyze the relations between sPLA2 activity and risk factors. Quartiles are defined according to sPLA2 activity distribution in controls.

Relationship between sPLA2 activity (as a continuous variable) and other continuous variables of cardiovascular risk is analyzed by calculating Pearson's correlation coefficients as well as the corresponding probability value.

Relative risks of coronary disease occurrence and their corresponding confidence intervals (95%) are calculated using a conditional logistic regression analysis taking into account age and sex matching.

The lower sPLA2 activity quartile is considered as a reference (RR=1).

Relative risks are adjusted on cardiovascular risk factors. Additional adjustments on other biological variables associated to coronary disease risk are provided.

Statistical analyses are carried out with the SAS version 8.2 software (SAS Institute, Cary, NC).

**Table 1. Association between plasma sPLA2 activity and base-line clinical variables**

| | | Means ±S.D of sPLA2 activity | Univariate P value | Multivariate P value |
|---|---|---|---|---|
| Recruitment center | Scotland | 3.1 ± 1.9 | <0.0001 | 0.0006 |
| | France | 2.4 ± 1.5 | | |

(continued)

|  | | Means ±S.D of sPLA2 activity | Univariate P value | Multivariate P value |
|---|---|---|---|---|
| Sex | M | 2.6 ± 1.6 | 0.4 | |
| | F | 2.8 ± 1.9 | | |
| Age | | * | <0.0001 | 0.002 |
| Medical history | | | | |
| Infarction | yes | 2.5 ± 1.4 | 0.3 | |
| | no | 2.7 ± 1.8 | | |
| Coronary artery disease | yes | 2.5 ± 1.4 | 0.15 | |
| | no | 2.7 ± 1.8 | | |
| Cerebrovascular disease | yes | 2.8 ± 1.3 | 0.6 | |
| | no | 2.6 ± 1.7 | | |
| Diabetes | yes | 3.0 ± 2.2 | 0.08 | |
| | no | 2.5 ± 1.6 | | |
| Hypertension | yes | 2.8 ± 1.8 | 0.07 | |
| | no | 2.5 ± 1.6 | | |
| Hyperlipidemia | yes | 2.4 ± 1.3 | 0.001 | 0.04 |
| | no | 2.9 ± 1.9 | | |
| PGI | yes | 2.3 ± 1.4 | 0.06 | |
| | no | 2.7 ± 1.7 | | |
| CABG | yes | 2.3 ± 1.3 | 0.3 | |
| | no | 2.7 ± 1.7 | | |
| Smoking status Current or former smoker | | 2.6 ± 1.66 | 0.3 | |
| | Never smoked | 2.8 ± 1.81 | | |
| Index diagnosis | | | | |
| Myocardial infarction with ST-segment elevation | | 3.0 ± 2.2 | 0.002 | 0.01 |
| Myocardial infarction without ST-segment elevation | | 2.4 ± 1.3 | | |
| Unstable angina | | 2.3 ± 1.4 | | |
| Killip class | 1 | 2.4 ± 1.6 | <0.0001 | 0.04 |
| | 2 | 3.6 ± 2.2 | | |
| | 3 | 3.2 ± 1.8 | | |
| Treatment within first 24 hrs hospital or chronic use | | | | |
| Aspirin | yes | 2.6 ± 1.7 | 0.2 | |
| | no | 2.3 ± 1.2 | | |
| Ticlopidine/ clopidogrel | yes | 2.5 ± 1.7 | 0.3 | |
| | no | 2.7 ± 1.7 | | |
| LMW Heparin | yes | 2.4 ± 1.4 | 0.001 | 0.03 |
| | no | 2.9 ± 2 | | |
| Unfractionated Heparin | yes | 2.9 ± 2 | 0.0008 | |
| | no | 2.3 ± 1.2 | | |
| GPIIb/IIIa | yes | 2.7 ± 1.9 | 0.6 | |
| | No | 2.6 ± 1.6 | | |
| Statin | yes | 2.5 ± 1.6 | 0.09 | |
| | No | 2.8 ± 1.9 | | |
| ACE inhibitor | yes | 2.8 ± 1.9 | 0.3 | |
| | No | 2.6 ± 1.6 | | |
| BetaBlocker | yes | 2.6 ± 1.8 | 0.5 | |

(continued)

| | Means ±S.D of sPLA2 activity | Univariate P value | Multivariate P value |
|---|---|---|---|
| No | 2.8 ± 1.5 | | |

Correlation coefficient with sPLA2 : 0.2

PCI : percutaneous intervention; CABG: coronary artery bypass graft; Killip class: classification of heart diseases (Braunwald (1992) Text Book of Cardiology 4th Ed); GPIIbIIIa: glycoprotein IIb/IIIa,

**Table 2. Base-line characteristics according to the tertile of sPLA2 activity**

| | Tertile 1 (0-1.7) | Tertile 2 (1.8-2.8) | Tertile 3 (≥2.9) | P Value |
|---|---|---|---|---|
| French recruitment center (%) | 80 | 59 | 50 | < 0.0001 |
| Male sex (%) | 79.3 | 77.9 | 74.5 | 0.6 |
| Age (Mean ± S.D.) | 57.2± 11.4 | 60.9 ±13.6 | 65.1 ±12.9 | <0.0001 |
| Medical history (%) | | | | |
| Infarction | 26.4 | 24.3 | 19.5 | 0.3 |
| Congestive heart failure | 2.9 | 2.9 | 10.1 | 0.02 |
| Coronary artery disease | 32.1 | 27.9 | 25.5 | 0.4 |
| Cerebrovascular disease | 5.0 | 5.1 | 6.7 | 0.8 |
| Diabetes | 16.4 | 12.5 | 21.5 | 0.11 |
| Hypertension | 35.7 | 42.6 | 43.6 | 0.3 |
| Hyperlipidemia | 60.7 | 49.3 | 40.3 | 0.003 |
| PCI | 22.9 | 13.2 | 12.1 | 0.007 |
| CABG | 8.0 | 9.2 | 3.9 | 0.17 |
| Current or former smoker (%) | 77.1 | 73.5 | 68.5 | 0.3 |
| Index diagnosis (%) | | | | |
| STEMI | 30.0 | 41.2 | 43.6 | |
| NSTEMI | 57.9 | 52.2 | 49.0 | 0.14 |
| Unstable angina | 12.1 | 6.6 | 7.4 | |
| Killip class > 1 (%) | 8.6 | 22.8 | 30.9 | < 0.0001 |
| Troponin I ±Mean ±S.D. | 9.8±24.2 | 24.6 ±68.5 | 28 ±54.7 | 0.006 |
| CRP ±Mean ±S.D. | 9.7 ±14.8 | 15.6 ±24.4 | 39.5 ±54.6 | < 0.0001 |
| IL18 ±Mean ±S.D. | 216.9 ± 87.6 | 227.5 ± 88.3 | 229.5 ±99.9 | 0.5 |
| Cholesterol ±Mean ±S.D. | 220.2 ±56 | 201.3 ±46 | 190.5 ±42.7 | < 0.0001 |

**Table 3. Multivariate analyses of the predictors of death, MI and death or MI**

| | R. R | 95% C.I | | p |
|---|---|---|---|---|
| **DEATH** | | | | |
| Medical history of CABG | 5.4 3 | 2.09 | 14.3 | 0.0005 |
| Killip class > 1 | 7.13 6 | 2.861 | 17.801 | <.0001 |
| sPLA2 tertile >=3 | 2.60 9 | 1.066 | 6.386 | 0.0358 |
| **MI** | | | | |
| Medical history of congestive heart failure | 6.0 2 | 1.77 | 20.4 | 0.004 |
| Creatinine >1.1 | 9.2 9, | 1.19 | 72.5 | 0.03 |
| sPLA2 tertile >=3 | 4. 7 | 1.27 | 17.5 | 0.02 |
| **DEATH or MI** | | | | |
| Killip class > 1 | 3.0 9 | 1.50 | 6.30 | 0.002 |

(continued)

| DEATH or MI | | | | |
|---|---|---|---|---|
| Age ≥ 60 yr | 2.9 6 | 1.12 | 7.83 | 0.03 |
| Medical history of CABG | 3.4 6 | 1.34 | 8.93 | 0.01 |
| Creatinine > 1.1 | 3.2 3 | 1.22 | 8.57 | 0.02 |
| sPLA2 activity, third tertile | 3.0 8 | 1.37 | 6.91 | 0.006 |

**Table 4. Sample characteristics**

| | |
|---|---|
| French recruitment center (%) | 63 |
| Male sex (%) | 76.6 |
| Age (Mean± S.D.) | 61.1+/-13 |
| Medical history (%) | |
| Infarction | 22.9 |
| Congestive heart failure | 5.6 |
| Coronary artery disease | 28.5 |
| Cerebrovascular disease | 5.6 |
| Diabetes | 16.6 |
| Hypertension | 40.2 |
| Hyperlipidemia | 49.6 |
| PCI | 16.4 |
| CABG | 6.9 |
| Current or former smoker (%) | 72.9 |
| Index diagnosis (%)<br>STEMI<br>NSTEMI<br>Unstable angina | <br>38.5<br>52.5<br>9.0 |
| Killip class > 1 (%) | 20.5 |
| Troponin I ng/mL (Median- Interquartile range) | 2.55-17.3 |
| CRP mg/L (Median-Interquartile range) | 8.45-15.7 |
| IL18 pg/mL (Median-Interquartile range) | 195-120 |
| Cholesterol mg/dL (Median- Interquartile range) | 197.4-70.4 |
| Creatinine mg/dL (Median-Interquartile range) | 1.1-0.26 |
| sPLA2 activity nmole/minlmL (Median-Interquartile range) | 2.3-1.78 |
| PLA2 Antigen ng/dL (Median-Interquartile range) | 279-459 |

**Table 5. Association between plasma sPLA2 activity and base-line clinical variables**

| | Means (S.D) of sPLA2 activity | Univariate P value | Multivariable P value |
|---|---|---|---|
| Recruitment center Scotland<br>France | 3.07 (1.9)<br>2.36 (1.5) | <0.000 | 0.0006 |

(continued)

|  |  | Means (S.D) of sPLA2 activity | Univariate P value | Multivariable P value |
|---|---|---|---|---|
| Sex M |  | 2.59 (1.6) | 0.4 |  |
| F |  | 2.77 (1.9) |  |  |
| Age |  | * | 0.4 | 0.002 |
| Medical history |  |  |  |  |
| Infarction | yes | 2.48 (1.4) | 0.3 |  |
|  | no | 2.67 (1.8) |  |  |
| Coronary artery disease | yes | 2.47(1.4) | 0.15 |  |
|  | no | 2.7 (1.8) |  |  |
| Cerebrovascular disease | yes | 2.79(1.3) | 0.6 |  |
|  | no | 2.63 (1.7) |  |  |
| Diabetes | yes | 3.03 (2.2) | 0.08 |  |
|  | no | 2.55 (1.6) |  |  |
| Hypertension | yes | 2.81 (1.8) | 0.07 |  |
|  | no | 2.51 (1.6) |  |  |
| Hyperlipidemia | yes | 2.36 (1.3) | 0.001 | 0.04 |
|  | no | 2.88 (1.9) |  |  |
| PCI | yes | 2.33 (1.4) | 0.06 |  |
|  | no | 2.69 (1.7) |  |  |
| CABG | yes | 2.33 (1.3) | 0.3 |  |
|  | no | 2.65 (1.7) |  |  |
| Smoking status Current or former smoker |  | 2.58 (1.7) | 0.3 |  |
| Never smoked |  | 2.77 (1.8) |  |  |
| Index diagnosis |  |  |  |  |
| Myocardial infarction with ST-segment elevation |  | 2.99 (2.2) | 0.002 | 0.01 |
| Myocardial infarction without ST-segment elevation |  | 2.42 (1.3) |  |  |
| Unstable angina |  | 2.31 (1.4) |  |  |
| Killip class |  |  |  |  |
| 1 |  | 2.43 (1.5) |  |  |
| 2 |  | 3.46 (2.2) | <0.0001 | 0.04 |
| 3 |  | 3.24 (1.8) |  |  |
| Treatment within first 24 hrs hospital or chronic use |  |  |  |  |
| Aspirin | yes | 2.65 (1.7) | 0.2 |  |
|  | no | 2.29 (1.2) |  |  |
| Ticlopidine / clopidogrel | yes | 2.54 (1.7) | 0.3 |  |
|  | no | 2.70 (1.7) |  |  |
| LMW Heparin | yes | 2.36 (1.4) | 0.001 |  |
|  | no | 2.90 (2.0) |  |  |
| Unfractionated Heparin | yes | 2.93 (2.0) | 0.0008 |  |
|  | no | 2.35 (1.2) |  | 0.03 |
| GPIIb/IIIa | yes | 2.71 (1.9) | 0.6 |  |
|  | no | 2.60 (1.6) |  |  |
| Statin | yes | 2.53 (1.6) | 0.09 |  |
|  | no | 2.84 (1.9) |  |  |
| ACE inhibitor | yes | 2.79 (1.9) | 0.3 |  |
|  | no | 2.59 (1.6) |  |  |
| Beta Blocker | yes | 2.62 (1.8) | 0.5 |  |

(continued)

| | | | | |
|---|---|---|---|---|
| Killip class | no | 2.77 (1.5) | | |

*Variables with a P<0.05 in univariate analysis were studied in multivariable analysis by performing an analysis of covariance. *Correlation coefficient with sPLA2: 0.2

### Table 6. Univariate survival analysis

| | | Cumulative Incidence of Death/MI (S.E)* | Relative Risk (95% confidence interval) | P |
|---|---|---|---|---|
| Recruitment center Scotland | | 10.5% (0.02) | 1.81 (0.93 - 3.53) | 0.08 |
| France | | 6.0% (0.01) | | |
| Sex | | | | |
| F | | 7.9% (0.03) | 1.13 (0.53-2.41) | 0.7 |
| M | | 7.7% (0.01) | | |
| Age | | | | |
| ≥60 yrs | | 13.2%(0.02) | 5.4 (2.26- 13) | <0.0001 |
| <60 yrs | | 2.3% (0.01) | | |
| Medical history | | | | |
| Infarction | yes | 14.9% (0.04) | 3.2 (1.65-6.13) | 0.0002 |
| | no | 5.6% (0.01) | | |
| Congestive heart failure | yes | 27.9% (0.09) | 4.41 (1.93-10.1) | 0.0001 |
| | no | 6.5% (0.01) | | |
| Coronary artery disease | yes | 12.1% (0.03) | 2.27 (1.18-4.36) | 0.01 |
| | no | 6.1% (0.01) | | |
| Cerebrovascular disease | yes | 16.0% (0.07) | 2.06 (0.73-5.83) | 0.16 |
| | no | 7.2% (0.01) | | |
| Diabetes | yes | 12.5% (0.04) | 1.95 (0.94-4.05) | 0.07 |
| | no | 6.8% (0.01) | | |
| Hypertension | yes | 10.2% (0.02) | 1.90 (1.01-3.55) | 0.05 |
| | no | 6.1% (0.01) | | |
| Hyperlipidemia | yes | 8.8% (0.02) | 1.44 (0.74-2.8) | 0.28 |
| | no | 6.8% (0.02) | | |
| PCI | yes | 4.4% (0.02) | 1.6 (0.57-4.52) | 0.37 |
| | no | 8.4% (0.01) | | |
| CABG | yes | 18.2% (0.07) | 2.67 (1.11-6.41) | 0.02 |
| | no | 6.9% (0.01) | | |
| Smoking status | | | | |
| Never smoked | | 10.6% (0.03) | 1.67 (0.86-3.27) | 0.13 |
| Current or former smoker | | 6.6% (0.01) | | |
| Index diagnosis | | | | |
| - MI with ST-segment elevation | | 9.9% (0.02) | 1.93 (0.96-3.86) | 0.06 |
| - other | | 5.7% (0.02) | | |
| Killip class | | | | |
| >1 | | 21.4% (0.04) | 5 (2.62 - 9.7) | <0.0001 |
| 1 | | 4.3% (0.01) | | |
| Creatinine | | | | |
| >1.1 | | 14% (0.02) | 6.2(2.39-15.95) | <0.0001 |

(continued)

| | Cumulative Incidence of Death/MI (S.E)* | Relative Risk (95% confidence interval) | P |
|---|---|---|---|
| ≤1.1 | 2% (0.01) | | |
| Total cholesterol | | | |
| ≤197 | 8.4% (0.02) | 1.34(0.59-3.06) | 0.5 |
| >197 | 5.2% (0.02) | | |
| Treatment | | | |
| LMW Heparin no | 8.7% (0.02) | 1.12 (0.57-2.2) | 0.7 |
| yes | 6.8% (0.02) | | |
| Unfractionated Heparin no | 8.4% (0.02) | 1.27 (0.64-2.52) | 0.5 |
| yes | 6.8% (0.02) | | |
| Revascularization no | 10.5% (0.02) | 2.42 (1.16-5.05) | 0.01 |
| (in hospital) yes | 4.3% (0.01) | | |
| Troponin I | | | |
| Tertile 1 | 6.2% (0.02) | R.R 2,3 vs 1 : 1.39 (0.59-3.30) | 0.5 |
| Tertile 2 | 7.8% (0.02) | R.R 3 vs 1,2 : 1.22 (0.57-2.61) | 0.6 |
| Tertile 3 | 9.4% (0.02) | | |
| CRP | | | |
| Tertile 1 | 7.7% (0.02) | R.R 2,3 vs 1 : 0.99 (0.43-2.3) | 0.9 |
| Tertile 2 | 7% (0.02) | R.R 3 vs 1,2 : 1.28 (0.58-2.81) | 0.5 |
| Tertile 3 | 8.8% (0.02) | | |
| IL18 | | | |
| Tertile 1 | 7.3% (0.02) | R.R 2,3 vs 1 : 0.99 (0.43-2.28) | 0.9 |
| Tertile 2 | 7% (0.02) | R.R 3 vs 1,2 : 1.31 (0.6-2.89) | 0.5 |
| Tertile 3 | 9.8% (0.02) | | |
| PLA2 Antigen | | | |
| Tertile 1 | 7.8% (0.02) | R.R 2,3 vs 1 : 1 (0.51-2) | 0.9 |
| Tertile 2 | 5% (0.02) | R.R 3 vs 1,2 : 1.9 (0.96-3.6) | 0.06 |
| Tertile 3 | 11.3% (0.03) | | |
| sPLA2 activity | | | |
| Tertile 1 | 3% (0.01) | R.R 2,3 vs 1 : 1.8 (0.5-6.2) | 0.3 |
| Tertile 2 | 5.1% (0.02) | R.R 3 vs 1,2 : 4.3 (2.1-8.7) | <0.0001 |
| Tertile 3 | 14.4% (0.03) | | |
| * 6 months cumulative incidence of death/MI (standard error). | | | |

**Table 7. Multivariable analysis of the predictors of death or MI**

| | R.R | 95% C.I | 95% C.I | P |
|---|---|---|---|---|
| **DEATH or MI** | | | | |
| Killip class > 1 | 3.09 | 1.50 | 6.30 | 0.00 2 |
| Age ,;:60 yr | 2.96 | 1.12 | 7.83 | 0.0 3 |
| Medical history of CABG | 3.46 | 1.34 | 8.93 | 0.0 1 |
| Creatinine > 1.1 | 3.23 | 1.22 | 8.57 | 0.0 2 |

(continued)

|  | R.R | 95% C.I | 95% C.I | P |
|---|---|---|---|---|
| **DEATH or MI** | | | | |
| sPLA2 activity, third tertile | 3.08 | 1.37 | 6.91 | 0.00 6 |
| Univariate predictors with a P<0.05 (Table 6) were included in a stepwise multivariable Cox regression model. The selection method was a forward selection with a significance level of 0.05 for entering an explanatory variable into the model. The final model only includes significant variables with a P<0.05 using the Wald test. | | | | |

**Table 8. Characteristics of a sample of 1400 control individuals and 700 case patients included in the EPIC-Norfolk 1993-2003 study**

|  | Controls | Cases |
|---|---|---|
| **Men** | | |
| Age | $64\pm8$ | $64\pm8$ |
| Smoker | | |
| No | 32% | 25% |
| Prior | 60% | 60% |
| Current | 8% | 15% |
| Body mass index ($Kg/m^2$) | $26\pm3$ | $27\pm4$ |
| Diabetes | 2.4% | 7.1 % |
| Systolic arterial pressure | $139\pm17$ (mmHg) | $144\pm18$ |
| Diastolic arterial pressure | $85\pm11$ | $87\pm11$ |
| Total cholesterol (mmol/l) | $6.1\pm1.1$ | $6.3\pm1.1$ |
| **Men** | | |
| LDL-cholesterol(nmol/l) | $4.0\pm1.0$ | $4.1\pm1.0$ |
| HDL-cholesterol (mmol/l) | $1.25\pm0.33$ | $1.16\pm0.31$ |
| Triglycerids (mmol/l) | 1.7 (1.2-2.4) | 2.0 (1.4-2.9) |
| C-reactive Protein (mg/ml) | 1.4 (0.7-2.9) | 2.2 (1.0-4.5) |
| **Women** | | |
| Age | $67\pm7$ | $67\pm7$ |
| Smoker | | |
| No | 55% | 45% |
| Prior | 37% | 40% |
| Current | 8% | 15%. |
| Body mass index ($Kg/m^2$) | $26\pm4$ | $27\pm5$ |
| Diabetes | 0.8% | 5.5% |
| Systolic arterial pressure | $139\pm18$(mmHg) | $143\pm19$ |
| Diastolic arterial pressure | $82\pm11$ | $85\pm12$ |
| Total cholesterol (mmol/l) | $6.6\pm1.2$ | $6.9\pm1.3$ |
| LDL-cholesterol(mmol/l) | $4.3\pm1.1$ | $4.5\pm1.1$ |
| HDL-cholesterol (mmol/l) | $1.59\pm0.4$ | $1.45\pm0.39$ |
| Triglycerids (mmol/l) | 1.5 (1.1-2.2) | 1.8 (1.3-2.6) |
| C-reactive Protein (mg/ml) | 1.6 (0.8-3.5) | 2.6 (1.1-5.8) |

**Claims**

1. A method for determining an increased risk of mortality due to a cardiac and/or vascular event or of myocardial infarction in a patient who has been diagnosed as being substantially healthy wherein substantially healthy meant that said patient presents no symptoms of affliction by a disease, in particular with respect to atherosclerosis, cardiac and/or vascular related diseases, comprising:

- determining the secretory Phospholipase A2 (sPLA2) activity in a biological sample of said patient,
- comparing said activity to a predetermined value, wherein the predetermined value corresponds to a sPLA2 activity comprised in the highest two tertiles of the sPLA2 activity range of a given population of substantially healthy individuals, in particular in the higher half, more particularly in the highest tertile of said range,

a higher sPLA2 activity of said patient as compared to said predetermined value being indicative of an increased risk of mortality due to a cardiac and/or vascular event or of myocardial infarction.

2. A method according to claim **1,** wherein no onset of ischemic symptoms has been diagnosed in the patient.

3. A method according to any of claims **1** to **2,** wherein the sPLA2 activity of the patient is higher than about 1.8 nmol/ml/min, in particular higher than about 2 nmol/ml/min, more particularly higher than about 2.5 nmol/ml/min, preferably higher than about 2.9 nmol/ml/min, and more preferably higher than 3.3.

4. A method according to any of claims **1** to **3,** wherein the sPLA2 activity is measured in a biological sample, in particular a serum or a plasma sample, and more particularly a heparinized plasma sample.

5. An in vitro or ex vivo method for the diagnosis of myocardial infarction in a patient who has been diagnosed as being substantially healthy wherein substantially healthy meant that said patient presents no symptoms of affliction by a disease, in particular with respect to atherosclerosis, cardiac and/or vascular related diseases, comprising:

- determining the sPLA2 activity of said patient,
- comparing said activity to a predetermined value, wherein the predetermined value corresponds to a sPLA2 activity comprised in the highest two tertiles of the sPLA2 activity range of a given population of substantially healthy individuals, in particular in the higher half, more particularly in the highest tertile of said range,

a higher sPLA2 activity of said patient as compared to said predetermined value being indicative of myocardial infarction.

6. An in vitro or ex vivo method according to claim **5,** wherein a higher sPLA2 activity of a patient as compared to a predetermined value is indicative of the affliction of said patient with myocardial infarction at the time the determination of the sPLA2 activity of said patient is made, or is indicative of a future affliction of said patient with myocardial infarction, in particular more than 72 hours after the time the determination of the sPLA2 activity of said patient is made.

7. An in vitro or ex vivo method according to claim **5** or **6,** wherein the sPLA2 activity of the patient is higher than about 1.8 nmol/ml/min, in particular higher than about 2 nmol/ml/min, more particularly higher than about 2.5 nmol/ml/min, preferably higher than about 2.9 nmol/ml/min, and more preferably higher than about 3.3 nmol/ml/min.

8. An in vitro or ex vivo method according to any of claims **5** to **7,** wherein the sPLA2 activity is measured in a biological sample, in particular a serum or a plasma sample, and more particularly a heparinized plasma sample.

**Patentansprüche**

1. Verfahren zur Bestimmung eines erhöhten Mortalitätsrisikos aufgrund eines kardialen und/oder vaskulären Ereignisses oder eines Herzinfarkts bei einem Patienten, der als im Wesentlichen gesund diagnostiziert wurde, wobei im Wesentlichen gesund bedeutete, dass der besagte Patient keine Symptome eines Leidens an einer Erkrankung, insbesondere in Bezug auf Atherosklerose, mit dem Herzen und/oder dem Gefäßsystem zusammenhängenden Erkrankungen aufweist, wobei das Verfahren Folgendes umfasst:

- Bestimmen der Aktivität sekretorischer Phospholipase A2 (sPLA2) in einer biologischen Probe des besagten Patienten,
- Vergleichen der besagten Aktivität mit einem vorherbestimmten Wert, wobei der vorherbestimmte Wert einer sPLA2-Aktivität entspricht, die von den höchsten zwei Tertilen des sPLA2-Aktivitätsbereichs einer gegebenen Population von im Wesentlichen gesunden Personen, insbesondere von der höheren Hälfte, ganz besonders von dem höchsten Tertil des besagten Bereichs umfasst wird,

wobei eine höhere sPLA2-Aktivität des besagten Patienten im Vergleich zu dem besagten vorherbestimmten Wert auf ein erhöhtes Mortalitätsrisiko aufgrund eines kardialen und/oder vaskulären Ereignisses oder eines Herzinfarkts hinweist.

2. Verfahren nach Anspruch **1,** wobei bei dem Patienten kein Einsetzen von ischämischen Symptomen diagnostiziert wurde.

3. Verfahren nach einem der Ansprüche **1** bis **2,** wobei die sPLA2-Aktivität des Patienten höher als etwa 1,8 nmol/ml/min, insbesondere höher als etwa 2 nmol/ml/min, ganz besonders höher als etwa 2,5 nmol/ml/min, vorzugsweise höher als etwa 2,9 nmol/ml/min und mehr bevorzugt höher als 3,3 ist.

4. Verfahren nach einem der Ansprüche **1** bis **3,** wobei die sPLA2-Aktivität in einer biologischen Probe, insbesondere einer Serum- oder einer Plasmaprobe und ganz besonders einer heparinisierten Plasmaprobe gemessen wird.

5. In-vitro- oder Ex-vivo-Verfahren zur Diagnose eines Herzinfarkts bei einem Patienten, der als im Wesentlichen gesund diagnostiziert wurde, wobei im Wesentlichen gesund bedeutete, dass der besagte Patient keine Symptome eines Leidens an einer Erkrankung, insbesondere in Bezug auf Atherosklerose, mit dem Herzen und/oder dem Gefäßsystem zusammenhängenden Erkrankungen aufweist, wobei das Verfahren Folgendes umfasst:

   - Bestimmen der sPLA2-Aktivität des besagten Patienten,
   - Vergleichen der besagten Aktivität mit einem vorherbestimmten Wert, wobei der vorherbestimmte Wert einer sPLA2-Aktivität entspricht, die von den höchsten zwei Tertilen des sPLA2-Aktivitätsbereichs einer gegebenen Population von im Wesentlichen gesunden Personen, insbesondere von der höheren Hälfte, ganz besonders von dem höchsten Tertil des besagten Bereichs umfasst wird,

   wobei eine höhere sPLA2-Aktivität des besagten Patienten im Vergleich zu dem besagten vorherbestimmten Wert auf einen Herzinfarkt hinweist.

6. In-vitro- oder Ex-vivo-Verfahren nach Anspruch **5,** wobei eine höhere sPLA2-Aktivität eines Patienten im Vergleich zu einem vorherbestimmten Wert auf das Leiden des besagten Patienten unter einem Herzinfarkt zu der Zeit, zu der die Bestimmung der sPLA2-Aktivität des besagten Patienten vorgenommen wird, hinweist oder auf ein künftiges Leiden des besagten Patienten unter einem Herzinfarkt, insbesondere mehr als 72 Stunden nach der Zeit, zu der die Bestimmung der sPLA2-Aktivität des besagten Patienten vorgenommen wird, hinweist.

7. In-vitro- oder Ex-vivo-Verfahren nach Anspruch **5** oder **6,** wobei die sPLA2-Aktivität des Patienten höher als etwa 1,8 nmol/ml/min, insbesondere höher als etwa 2 nmol/ml/min, ganz besonders höher als etwa 2,5 nmol/ml/min, vorzugsweise höher als etwa 2,9 nmol/ml/min und mehr bevorzugt höher als etwa 3,3 nmol/ml/min ist.

8. In-vitro- oder Ex-vivo-Verfahren nach einem der Ansprüche **5** bis **7,** wobei die sPLA2-Aktivität in einer biologischen Probe, insbesondere einer Serum- oder einer Plasmaprobe und ganz besonders einer heparinisierten Plasmaprobe gemessen wird.

## Revendications

1. Procédé pour déterminer un risque accru de mortalité due à un évènement cardiaque et/ou vasculaire ou à un infarctus du myocarde chez un patient qui a été diagnostiqué comme étant substantiellement en bonne santé, dans lequel substantiellement en bonne santé signifie que ledit patient ne présente aucun symptôme d'atteinte par une maladie, en particulier au regard de l'athérosclérose, des maladies cardiaques et/ou vasculaires, comprenant :

   - la détermination de l'activité Phospholipase A2 sécrétoire (sPLA2) dans un échantillon biologique dudit patient,
   - la comparaison de ladite activité à une valeur prédéterminée, dans lequel la valeur prédéterminée correspond à une activité sPLA2 comprise dans les deux plus hauts tertiles de la gamme d'activité sPLA2 d'une population donnée d'individus substantiellement en bonne santé, en particulier dans la plus haute moitié, plus particulièrement dans le plus haut tertile de ladite gamme,

   une activité sPLA2 plus élevée dudit patient comparée à ladite valeur prédéterminée étant indicative d'un risque accru de mortalité due à un évènement cardiaque et/ou vasculaire ou à un infarctus du myocarde.

**2.** Procédé selon la revendication **1,** dans lequel aucune apparition de symptômes ischémiques n'a été diagnostiquée chez le patient.

**3.** Procédé selon l'une quelconque des revendications **1** à **2,** dans lequel l'activité sPLA2 du patient est supérieure à environ 1.8 nmol/ml/min, en particulier supérieure à environ 2 nmol/ml/min, plus particulièrement supérieure à environ 2.5 nmol/ml/min, préférentiellement supérieure à environ 2.9 nmol/ml/min, et plus préférentiellement supérieure à 3.3.

**4.** Procédé selon l'une quelconque des revendications **1** à **3,** dans lequel l'activité sPLA2 est mesurée dans un échantillon biologique, en particulier un échantillon de sérum ou de plasma, et plus particulièrement un échantillon de plasma hépariné.

**5.** Procédé *in vitro* ou *ex vivo* pour le diagnostic de l'infarctus du myocarde chez un patient qui a été diagnostiqué comme étant substantiellement en bonne santé, dans lequel substantiellement en bonne santé signifie que ledit patient ne présente aucun symptôme d'atteinte par une maladie, en particulier au regard de l'athérosclérose, des maladies cardiaques et/ou vasculaires, comprenant :

- la détermination de l'activité sPLA2 dudit patient,
- la comparaison de ladite activité à une valeur prédéterminée, dans lequel la valeur prédéterminée correspond à une activité sPLA2 comprise dans les deux plus hauts tertiles de la gamme d'activité sPLA2 d'une population donnée d'individus substantiellement en bonne santé, en particulier dans la plus haute moitié, plus particulièrement dans le plus haut tertile de ladite gamme,

une activité sPLA2 plus élevée dudit patient comparée à ladite valeur prédéterminée étant indicative d'un infarctus du myocarde.

**6.** Procédé *in vitro* ou *ex vivo* selon la revendication **5,** dans lequel une activité sPLA2 plus élevée d'un patient comparée à une valeur prédéterminée est indicative de l'atteinte dudit patient par un infarctus du myocarde au moment où la détermination de l'activité sPLA2 dudit patient est faite, ou est indicative d'une future atteinte dudit patient par un infarctus du myocarde, en particulier plus de 72 heures après le moment où la détermination de l'activité sPLA2 dudit patient est faite.

**7.** Procédé *in vitro* ou *ex vivo* selon les revendications **5** ou **6,** dans lequel l'activité sPLA2 du patient est supérieure à environ 1.8 nmol/ml/min, en particulier supérieure à environ 2 nmol/ml/min, plus particulièrement supérieure à environ 2.5 nmol/ml/min, préférentiellement supérieure à environ 2.9 nmol/ml/min, et plus préférentiellement supérieure à environ 3.3 nmol/ml/min.

**8.** Procédé *in vitro* ou *ex vivo* selon l'une quelconque des revendications **5** à **7,** dans lequel l'activité sPLA2 est mesurée dans un échantillon biologique, en particulier un échantillon de sérum ou de plasma, et plus particulièrement un échantillon de plasma hépariné.

**Figure 1**

**Figure 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DENNIS.** *J Biol Chem.,* 1994, vol. 269, 13057-13060
- **ELINDER et al.** *Arterioscler Thromb Vase Biol.,* 1997, vol. 17, 2257-63
- **MANGIN et al.** *Circ Res.,* 1993, vol. 72, 161-6
- **LEITINGER et al.** *Arterioscler Thromb Vase Biol.,* 1999, vol. 19, 1291-8
- **KUGIYAMA et al.** *Circulation,* 1999, vol. 100, 1280-4
- **LIU et al.** *Eur Heart J.,* 2003, vol. 24, 1824-32
- **KUGIYAMA et al.** *Am J Cardial.,* 2000, vol. 86, 718-22
- **LANGTON ; JARNICKI.** *Clin. Chim. Acta,* 1992, vol. 205, 223-231
- **SCHAEFER et al.** *J. Clin. Chem.,* 1990, vol. 28, 253-254
- **RADVANYI et al.** *Anal Biochem.,* 1989, vol. 177, 103-9
- **PERNAS et al.** *Biochem. Biophys. Res. Commun.,* 1991, vol. 178, 1298-1305
- **STEG et al.** *Circulation,* 2004, vol. 109, 494-9
- **RADVANYI et al.** *Anal. Biochem.,* 1989, vol. 177, 103-9
- **EAGLE et al.** *Jama,* 2004, vol. 291, 2727-33
- **LIUZZO et al.** *N Engl J Med.,* 1994, vol. 331, 417-24
- **MORROW et al.** *J Am Coll Cardiol.,* 1998, vol. 31, 1460-5
- **HEESCHEN et al.** *J Am Coll Cardiol.,* 2000, vol. 35, 1535-42
- **LINDAHL et al.** *N Engl J Med.,* 2000, vol. 343, 1139-47
- **JAMES et al.** *Circulation,* 2003, vol. 108, 275-81
- **MUELLER et al.** *Circulation,* 2002, vol. 105, 1412-5
- **ZAIRIS et al.** *Am Heart J.,* 2002, vol. 144, 782-9
- **BENNERMO et al.** *J Intern Med.,* 2003, vol. 254, 244-50
- **BENAMER et al.** *Am J Cardiol.,* 1998, vol. 82, 845-50
- **MALLAT et al.** *Circ Res.,* 2001, vol. 89, E41-5
- **WHITMAN et al.** *Circ Res.,* 2002, vol. 90, E34-8

- **BLANKENBERG et al.** *Circulation,* 2002, vol. 106, 24-30
- **BLANKENBERG et al.** *Circulation,* 2003, vol. 108, 2453-9
- **KUGIYAMA et al.** *Am J Cardiol.,* 2000, vol. 86, 718-22
- **PRUZANSKI ; VADAS.** *Immunol Today,* 1991, vol. 12, 143-6
- **MURAKAMI et al.** *J Lipid Mediat Cell Signal.,* 1995, vol. 12, 119-30
- **HURT-CAMEJO et al.** *Arterioscler Thromb Vase Biol.,* 1997, vol. 17, 300-9
- **PRUZANSKI ; VADAS.** *Immunol Today.,* 1991, vol. 12, 143-6
- **NAKANO ; ARITA.** *FEBS Lett.,* 1990, vol. 273, 23-6
- **MURAKAMI et al.** *J Biol Chem.,* 1993, vol. 268, 839-44
- **KUWATA et al.** *J Biol Chem.,* 1998, vol. 273, 1733-40
- **IVANDIC et al.** *Arterioscler Thromb Vase Biol.,* 1999, vol. 19, 1284-90
- **FOURCADE et al.** *Cell.,* 1995, vol. 80, 919-927
- **SWARTHOUT ; WALLING.** *Cell Mol Life Sci.,* 2000, vol. 57, 1978-85
- **KUME et al.** *J Clin Invest.,* 1992, vol. 90, 1138-44
- **MENSCHIKOWSKI et al.** *Atherosclerosis,* 1995, vol. 118, 173-81
- **HURT-CAMEJO et al.** *Artérioscler Thromb Vase Biol.,* 1997, vol. 17, 300-9
- **FOURCADE et al.** *Adv Enzyme Regul.,* 1998, vol. 38, 99-107
- **MALLAT et al.** *Circulation,* 1999, vol. 99, 348-353
- **MALLAT et al.** *Circulation,* 2000, vol. 101, 841-3
- **DAY N et al.** EPIC-Norfolk: study design and characteristics of the cohort. *European Prospective Investigation of Cancer. Br J Cancer,* 1999, vol. 80 (11), 95-103
- International Classification of Diseases
- **BRAUNWALD.** Text Book of Cardiology. 1992